# EUROPÄISCHE PATENTSCHRIFT

(11) **EP 2 216 301 B1**
(45) Veröffentlichungstag und Bekanntmachung des Hinweises auf die Patenterteilung: **24.10.2012**
(21) Anmeldenummer: 10000932.3
(22) Anmeldetag: 29.01.2010
(51) Int. Cl.: C02F 3/30, B09B 3/00, C05F 17/00, C12M 1/107

(54) **Verfahren zur Behandlung von bei der Trockengärung anfallendem Perkolatwasser**
Method for treating percolate water accumulated during dry cooking
Procédé de traitement d'eau de percolateur produite dans la fermentation sèche

(30) Priorität: 10.02.2009 DE 102009008254
(43) Veröffentlichungstag der Anmeldung: 11.08.2010
(73) Patentinhaber: Holm, Niels Christian, 32425 Minden (DE)
(72) Erfinder: Holm, Niels Christian, 32425 Minden (DE)
(74) Vertreter: Lobemeier, Martin Landolf

(56) Entgegenhaltungen:
- EP-A1- 1 736 535
- WO-A1-2007/012328
- DE-A1- 4 409 487
- DE-U1-202006 003 293
- IT-B- 1 244 862
- JP-A- 2005 238 185
- US-A- 5 269 634

## Beschreibung

Die Erfindung betrifft ein Verfahren zur biologischen Behandlung von Perkolatwasser, das bei der Trockenvergärung von Bioabfällen oder sonstigen festen organischen Stoffen entsteht.

Bioabfälle ohne nennenswerte Störstoffe werden heutzutage überwiegend als Ersatzfuttermittel genutzt oder in Biogasanlagen oder kommunalen Faulbehälteranlagen energetisch verwertet, wobei die Verarbeitzug von Bioabfällen oder nachwachsenden Rohstoffen in erster Linie von der Zusammensetzung des jeweiligen Materials abhängt.

Bioabfälle mit bedeutenden Anteilen an Störstoffen, z.B. organische Haushaltsabfälle, wie sie in Deutschland in den so genannten Biotonnen anfallen, werden üblicherweise in mechanisch biologischen Abfallbehandlungsanlagen (MBA) verarbeitet. Dabei wird nach mehreren unterschiedlichen Vorbehandlungsschritten und üblicherweise eines letzten Kompostierungsschrittes der organische Anteil in diesen Bioabfällen so weit reduziert bzw. stabilisiert, dass eine nachfolgende Deponierung oder Verwertung als Kompost möglich ist.

Ein Verfahren und eine Anlage zur Vergärung von biogen-organischen Rohabfällen ist beispielsweise aus der DE 44 09 487 A1 bekannt.

Vor der Kompostierung können die Abfallfraktionen mit einem ausreichend hohen Anteil an organischen Inhaltstoffen einer energetischen Verwertung in Form einer Trockenvergärung in speziellen Fermentern zugeführt werden. Dabei wird ein Großteil der organischen Fraktionen (nachfolgend als CSB = Chemischer Sauerstoff Bedarf bezeichnet) unter anaeroben Bedingungen zu Biogas vergoren. Die Vergärung wird mittels der so genannten Perkolatwasserrezirkulation in Gang gesetzt bzw. beschleunigt und optimiert: Im Verlaufe des Gärprozesses sickert hochbelastetes Abwasser (das so genannte Perkolatwasser) durch die Bioabfallmiete, wird im Bodenbereich gesammelt und einem Perkolatwasserbehälter zugeführt. Aus diesem erfolgt dann die Rezirkulation mittels Berieselung der Bioabfallmiete mit dem Perkolatwasser. Je nach Berieselungsart und -Intensität erfolgt die Biogasproduktion überwiegend im Perkolatwasserbehälter oder in der Bioabfallmiete, was für die vorliegende Erfindung jedoch ohne Bedeutung ist.

In den meisten Fällen entsteht in Abhängigkeit vom Wassergehalt der behandelten Bioabfälle eine mehr oder weniger große Überschussmenge an hochbelastetem Perkolatwasser. Nur wenn dieser Wassergehalt eine bestimmte Mindestgrenze unterschreitet, entsteht kein Perkolatwasserüberschuss, der entsorgt werden muss.

Ansonsten muss der entstehende Perkolatwasserüberschuss weiterbehandelt und/oder entsorgt werden. Da dieser Perkolatwasserüberschuss mit organischen Inhaltstoffen (CSB oft über 20.000 mg/l) und mit Stickstoffverbindungen hoch belastet ist, ist die Weiterbehandlung bzw. Entsorgung technisch sehr aufwendig und teuer. Die bisherigen Weiterbehandlungsverfahren erfolgen extern ohne Verbindung mit der vorgeschalteten anaeroben Bioabfallvergärung.

Je nach Zusammensetzung der zu behandelnden Bioabfälle entsteht ein Perkolatwasser, das Hemmstoffe enthalten kann, die die Biogasbildung hemmen können, beispielsweise durch Freisetzung bzw. Aufkonzentrierung hoher Ammoniakmengen. Des Weiteren sind im Perkolatwasserüberschuss noch hohe Mengen an organischen Restsubstanzen vorhanden, deren Biogasbildungspotential ungenutzt bleibt. Wenn nach der Anaerobbehandlung der Bioabfall (inklusive des Perkolatwassers) aus den Fermentern entnommen wird, erfolgt daher noch eine bedeutende Kohlendioxid- und Methanemission in die Atmosphäre, was vor dem Hintergrund der Klimaproblematik äußerst negativ zu bewerten ist. Letztlich ist der ausgegorene Bioabfall mit dem Rohperkolatwasser gesättigt, was die Weiterverarbeitung und/oder Entsorgung dieses ausgegorenen Bioabfalles erschwert bzw. verteuert. Beispielsweise würde bei einem nachfolgenden Kompostierungsschritt hochbelastetes Sickerwasser entstehen.

Aufgabe dieser Erfindung ist es deshalb, ein Verfahren bereit zu stellen, bei dem die Belastung mit Perkolatwasser weitgehend vermieden wird.

Diese Aufgabe wird durch dass Verfahren mit den in Anspruch 1 genannten Schritten gelöst. Die Unteransprüche geben vorteilhafte Ausgestaltungen der Erfmdung wieder.

Dieses Verfahren hat die folgenden Vorteile:
a) Die Belastung des Perkolatwasserüberschusses wird so weitgehend reduziert wird, dass die Entsorgungskosten dieses weiterbehandelten Perkolatwasserüberschusses stark reduziert werden. Das geschieht beispielsweise dadurch, dass das Perkolatwasser auf Indirekteinleiterqualität gereinigt wird.
b) Der zu behandelnde Perkolatwasserüberschuss wird in die Perkolatrezirkulation der Trockenvergärung dergestalt integriert, dass die Biogasbildung durch Ausnutzung der Restorganik und Minderung der Hemmwirkung des Rohperkolatwassers erhöht wird.
c) Die Qualität des ausgegorenen Bioabfalls wird so sehr verbessert, dass dessen Weiterbehandlung und/oder Entsorgung weniger Kosten verursacht,
d) die Kohlendioxid- und Methanemissionen in die Atmosphäre werden erheblich reduziert.

Die Erfindung wird im Folgenden anhand einer Zeichnung erläutert. Dabei zeigt:
Fig. 1 eine schematische Darstellung eines vorgeschalteten Denitrifikationsverfahren, und
Fig. 2 eine schematische Darstellung der Beschickungen der Perkolatwasserbehälter und der Fermenterkammern.

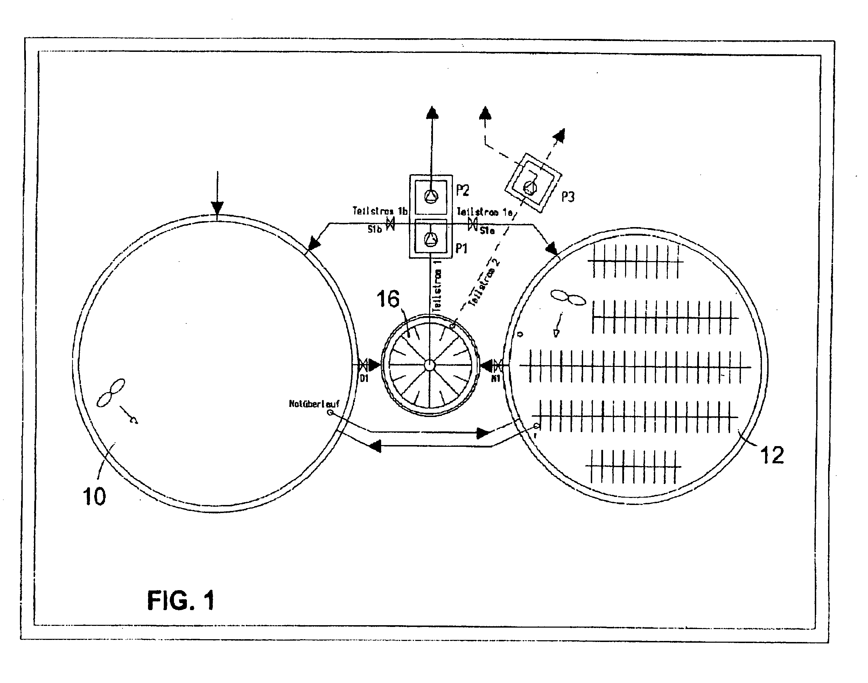

Fig. 1 zeigt, dass das Perkolatwasser (aus dem oder einem der in Fig. 1 nicht dargestellten Perkolatwasserbehälter) in ein vorgeschaltetes Denitrifikationsbecken 10 gefördert wird. In dieses Denitrifikationsbecken 10 wird weiter über eine interne Rezirkulation Belebtschlamm aus einem nachgeschalteten Nitrifikationsbecken 12 gefördert (Notüberlauf 14 von Nitrifikationsbecken 12 in das Denitriflkationsbecken 10). In dem Denitrifikationsbecken 10 erfolgt die Denitrifikation des im Nitrifikationsbecken (12) gebildeten Nitrates mit Hilfe der aus dem Perkolatwassers zugeführten Kohlenstoffquellen.

Die Weiterleitung in das Nitrifikationsbecken 12 erfolgt nicht wie üblich direkt, sondern über ein zwischengeschaltetes Absetzbecken 16, das hier beispielhaft als vertikal durchströmtes Trichterbecken (Dortmundbrunnen) dargestellt ist. Dafür wird die Verschlussarmatur D1 geöffnet und die Verschlussarmatur N1 geschlossen. Aus der Spitze des Absetzbeckens 16 wird kontinuierlich ein Teilstrom 1 entnommen und über ein Pumpwerk P1 in das Nitrifikationsbecken 12 geleitet (Teilstrom 1a); dafür wird die Verschlussarmatur S1a geöffnet und die Verschlussarmatur S1b geschlossen. Der verbleibende Teilstrom 2 läuft als Überstand, in dem kein oder nur eine geringe Belebtschlammmenge vorhanden ist, in das Pumpwerk P3 ab. Dieser Teilstrom 2, in dem ein Teil oder der ganze gebildete Überschuss-Schlamm dieser Belebtschlammstufe enthalten ist, wird in die Perkolatwasserrezirkulation der vorgeschalteten anaeroben Trockenvergärung zurückgeführt und somit integriert.

Die Rezirkulation zwischen Denitrifikations- und Nitrifikationsbecken kann umgekehrt erfolgen: Dafür werden lediglich D1 und S1a geschlossen und N1 und S1b geöffnet. Dann erfolgt die Rezirkulation aus dem Nitrifikationsbecken in das Denitrifikationsbecken. Die jeweiligen Rückläufe sind in Fig.1 dargestellt. Bedeutsam ist hierbei, dass bei dieser Prozessführung der Teilstrom 2 aus dem Nitrifikationsbecken 12 stammt und somit gar kein Ammonium mehr enthält und hinsichtlich der gelösten Kohlenstoffverbindungen weitestgehend oxidiert ist. Dieses Wasser eignet sich daher noch besser für eine besonders weitgehende Perkolatwasserauswaschung aus den vergärenden Bioabfällen.

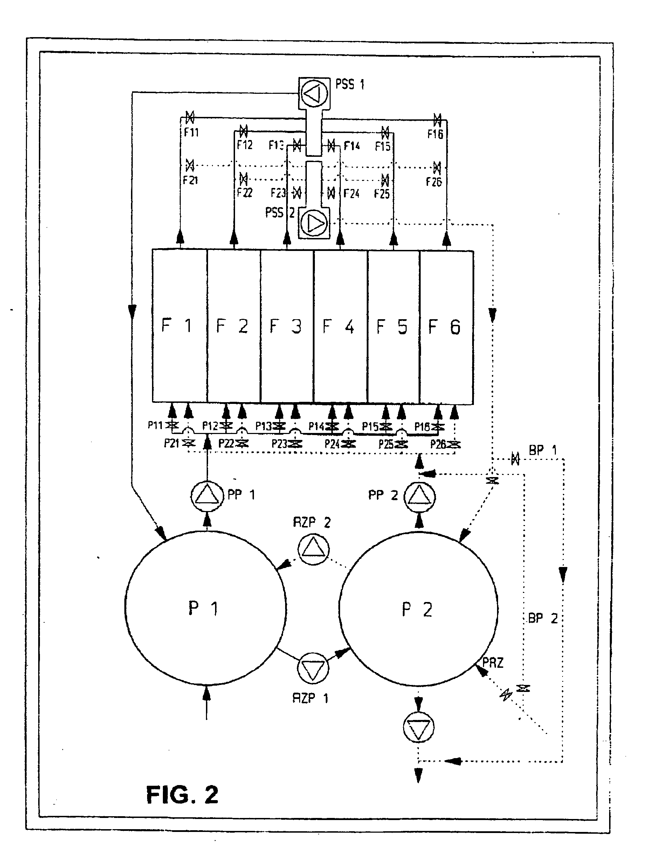

Weiter kann, wie Fig. 1 verdeutlicht, der gebildete Überschuss-Schlamm der aeroben Biologie in die Trockenvergärung integriert sein: Mittels des Pumpwerks P2 kann der Überschuss-Schlamm direkt in einen (in Fig. 2 dargestellten) Perkolatwasserbehälter gefördert werden. Mit diesem neuartigen Verfahrensschritt zwischen Denitrifikations- und Nitrifikationsbecken werden folgende Prozessvorteile erreicht:
1. Es erfolgt eine extreme Schlammindexselektion (der Schlammindex ISV ist ein Maß für die Absetzgeschwindigkeit des Belebtschlammes) in Richtung eines geringen Schlammindexes, da der Teilstrom 1, der im System verbleibt, einen überproportional hohen Anteil an schnell absetzbaren Flocken enthält. Diese Schlammindexselektion basiert auf dem gleichen Prinzip anderer Schlammindexselektionsverfahren (EP 1 634 854, EP 1 634 855 und EP 1 627 854), die technische Realisierung ist allerdings anders.

Auf Basis dieser ISV Selektion in Richtung eines sehr niedrigen ISV's können das Nitrifikationsbecken 10 und das Denitrifikationsbecken 12 mit einer vergleichsweise sehr hohen Belebtschlammkonzentration von über 10 g TS/1 betrieben werden, was entsprechend kleinere Beckenvolumina ermöglicht.
2. Der Teilstrom 2 wird als vorbehandeltes Perkolatwasserrezirkulat (mit stark reduzierten Stickstoff und Kohlenstoff (= CSB) Konzentrationen) in den Perkolatwasserbebälter der Trockenfermentation oder direkt in eine der Fermentationskammern zurückgeleitet, um eine über das Normalmaß hinausgehende Perkolatwasserauswaschung zu erreichen. Damit wird die Biogasproduktion optimiert und die Methanemissionen in die Atmosphäre minimiert.

Auch der Perkolatwasserüberschuss wird zur Indirekteinleitung oder Weiterbehandlung dem Teilstrom 2 entnommen. Bevorzugt erfolgt diese Entnahme bei Realisierung von Teilstrom 1a, weil dann der Teilstrom 2 das am weitestgehend gereinigte Wasser aus dem Nitrifikationsbecken ist.
3. Die wahlweise Realisierung von Teilstrom 1a und Teilstrom 1b kann folgendermaßen zur Prozessoptimierung genutzt werden: Am Beginn der Perkolatwasserauswaschung aus den vergärenden Bioabfällen wird 1a realisiert. Das beinhaltet einen schonenden Auswaschungsbeginn ohne nennenswerte Hemmung der Biogasproduktion, da der Teilstrom 2 dann aus dem Denitrifikationsbecken 10 stammt. Am Ende der Perkolatwasserauswaschung sollte diese durch Realisierung von 1b intensiviert werden. Dann ist der Teilstrom 2 (dann aus dem Nitrifikationsbecken) sehr weitgehend gereinigt und die Perkolatwasserauswaschung kann bis zur völligen Auswaschung der Restorganik vervollständigt werden.
4. Der aus der Spitze des Trichters 16 kontinuierlich entnommene Teilstrom 1 kann (manuell oder vollautomatisch) so weitgehend erhöht werden, dass der Teilstrom 2 frei von Belebtschlamm ist. Es ist besonders am Ende der Trockenfermentationsphase von unmittelbar erkennbarem Vorteil, wenn die Perkolatwasserrezirkulation frei von Belebtschlammbestandteilen ist.

Es wird jetzt auf Fig. 2 bezug genommen. Die eigentliche Trockenvergärung kann mit zwei Perkolatwasserbehältern ausgeführt werden. Die Zufuhr in das Perkolatwasserberieselungssystem wird auch für alle Fermentationskammern F1 - F6 redundant ausgeführt. Es wird jeweils eine Zufuhr einem Perkolatwasserbehälter P1, P2 zugeordnet. Aus jedem Perkolatwasserbehälter können wahlweise unterschiedliche Fermentationskammern beschickt werden. Diese Redundanz gilt auch für die Rückführung der Perkolatsickerwässer.

Zwischen Perkolatwasserbehälter P1 und allen Fermentationskammern F1 - F6 bis auf der, deren Prozess als nächstes abgeschlossen ist, erfolgt die "übliche" Perkolatwasserrezirkulation mit Rohperkolatwasser wie bei den bisher bekannten Trockenvergärungs-Perkolations-Systemen.

Der Perkolatwasserbehälter P2 kann der Perkolatwasserrezirkulation mit der Fermentationskammer, die als nächstes entleert wird, dienen; auf Grund der redundant ausgeführten Rückführleitungen wird gewährleistet, dass das Perkolatsickerwasser aus der Fermentationskammer, die aus dem Perkolatwasserbehälter P2 berieselt wird, auch in diesen zurückgeführt wird. Des Weiteren erfolgt aus diesem Perkolatwasserbehälter P2 oder der Bypassleitung BP1 die Rezirkulation in die Denitrifikation der aeroben Perkolatwasserbehandlung.

In Fig. 2 sind die beiden Perkolatwasserbehälter P1, P2 und sechs Fermentationskammern F 1 - F6 vorhanden. Jede Fermentationskammer F1 - F6 wird beispielsweise alle 6 Wochen entleert und mit frischem Material gefüllt. Diese Beschickungen/Entleerungen erfolgen mit jeweils einer Woche Versatz, so dass jede Woche eine Entleerung und Beschickung erfolgt.

Mit Beginn von Woche 1 wird die Fermentationskammer F1 (die eine sechswöchige anaerobe Vergärung hinter sich hat) geöffnet und entleert und anschließend neu beschickt. Bis zum Öffnen ist diese Kammer 1 die vorangegangene ganze Woche aus dem Perkolatwasserbehälter P2 (oder direkt aus dem Teilstrom 2 Abläufen des Nitrifikationsbeckens 12 oder des Denitrifikationsbeckens 10) berieselt worden. Nach Öffnung von Kammer F1 wird die Berieselung aus dem Perkolatwasserbehälter P2 auf die Kammer F2 umgestellt bis zum Beginn von Woche 2 usw. Alle anderen Fermentationskammern F1 - F6, die nicht aus dem Perkolatwasserbehälter P2 berieselt werden, werden aus dem Perkolatwasserbehälter P1 berieselt.

In Tab. 1 ist dieser Vorgang in einem Zyklusstrategiebild dargestellt*):

| Woche | **1** | **2** | **3** | **4** | **5** | **6** | **7** | **8** | **9** |
|---|---|---|---|---|---|---|---|---|---|
| F1 aus P1 | x | x | x | x | x | | x | x | X |
| F1 aus P2 | | | | | | x | | | |
| F2 aus P1 | | x | | x | x | x | | x | X |
| F2 aus P2 | x | | | | | | x | | |
| F3 aus P1 | x | | | x | x | x | x | | X |
| F3 aus P2 | | x | | | | | | x | |
| F4 aus P1 | x | x | | x | x | x | x | x | |
| F4 aus P2 | | | x | | | | | | X |
| F5 aus P1 | x | x | | | x | x | x | x | X |
| F5 aus P2 | | | | x | | | | | |
| F6 aus P1 | x | x | x | x | | x | x | x | X |
| F6 aus P2 | | | | | x | | | | |

| | | | | | | | | | |
|---|---|---|---|---|---|---|---|---|---|
| *) x in Woche 1 bei F1 aus P1 bedeutet, dass in dieser Woche die Fermentationskammer F1 mit dem Perkolatwasserbehälter P1 rezirkuliert wird. x in Woche 1 bei F2 aus P2 bedeutet, dass in dieser Woche die Fermentationskammer F 2 mit dem Perkolatwasserbehälter P2 (oder Teilstrom 2, siehe Bild 1) rezirkuliert wird. Zu erkennen ist, dass mit jeder neuen Woche die nächste Fermentationskammer dem Perkolatwasserbehälter P2 zugeordnet wird. | | | | | | | | | |

In Fig. 2 ist der Gesamtprozess der Trockenvergärung inklusive Perkolation schematisch dargestellt: Über den Perkolatwasserbehälter P 2 können über Verschlussarmaturen P21 bis P26 alle sechs Fermenterkammern F1 - F6 einzeln beschickt werden. Unter Normalbedingungen ist nur eine dieser Verschlussarmaturen P21 - P26 geöffnet, und zwar die, die dem Fermenter zugeordnet ist, der als nächstes entleert wird (beispielhaft in der Fig. 2 die Verschlussarmatur P22 analog Tab. 1). Die anderen fünf Fermenterkammern (F1 und F3 - F 6) werden über das Rezirkulationspumpwerk PP 1 aus dem Perkolatbehälter P1 beschickt. Damit korrespondierend werden die Sickerwässer aus den sechs Fermentern differenziell in die beiden Perkolatsammelschächte PSS 1 (P1 zugeordnet) und PSS 2 (P2 zugeordnet) geführt. Diese Korrespondenz erfolgt über Verschlussarmaturen F11 bis F26: Nur F22, also die Verbindung von der Fermenterkammer F2 zu dem Perkolatwasserschacht PSS 2 ist geöffnet, damit das Perkolat aus F2 zurück in den Perkolatwasserbehälter P2 rezirkuliert oder über Bypass BP1 direkt der aeroben Nachbehandlung (siehe Bild 1) zugeführt wird.

Der mit dem Perkolatwasserbehälter P1 in Verbindung stehende Perkolatwasserüberschuss kann gänzlich oder teilweise aus dem Perkolatsickerwasser des mit dem Perkolatwasserbehälter P1 in Verbindung stehenden Fermenters entnommen werden, der als nächstes den Perkolatwasserbehälter P2 zugeordnet wird. In diesem Beispiel ist das die Fermenterkammer F3. Von allen mit dem Perkolatwasserbehälter P1 in Verbindung stehenden Fermenterkammern F1 - F6 ist in der Fermenterkammer F3 der Vergärungsprozess am weitesten fortgeschritten und das Perkolatsickerwasser somit am geringsten belastet. Diese Zuordnung einer zweiten Fermenterkammer zu dem Perkolatwasserschacht PSS 2 kann beispielsweise über eine Soll-Höhenstandsvorgabe in dem Perkolatwasserbehälter P1 erfolgen: Wenn diese Sollhöhe überschritten ist, wird die Verschlussarmatur F23 geöffnet und die Verschlussarmatur F13 geschlossen, bis eine frei wählbare Menge aus dem Perkolatwasserbehälter P1 entnommen worden ist.

Die Verschlussarmaturen der anderen Fermenterkammer F 4 bzw. F 5 sind in Richtung auf den Perkolatwasserschacht PSS 1 geöffnet, von wo aus die Rezirkulation in den Perkolatwasserbehälter P1 erfolgt.

Nach Öffnung und Entleerung von F 2 wird die Verschlussarmatur P22 geschlossen und die Verschlussarmatur P23 geöffnet sowie die Verschlussarmatur F22 geschlossen und die Verschlussarmatur F23 geöffnet.

Die 4 oder 5 mit dem Perkolatwasserbehälter P1 verbundenen Fermenter produzieren kontinuierlich einen Perkolatwasserüberschuss. Dieser wird ganz oder teilweise über ein Pumpwerk oder Überlauf RZP1 in den Perkolatwasserbehälter 2 gefördert, wenn dieser Überschuss nicht über das Perkolatsickerwasser des im Vergärungsprozess am weitesten fortgeschrittenen, mit der Perkolatwasserbehälter P 1 in Verbindung stehenden Fermenterkammer F 1 - F 6 erfolgt.

Der in dem Perkolatwasserbehälter P1 entstehende Perkolatwasserüberschuss ist genau um die Menge an Überschuss-Schlamm aus der nachgeschalteten aeroben Biologie erhöht, die nach einer bevorzugten Ausgestaltung der Erfindung in den Perkolatwasserbehälter P1 gefördert wird.

Vorzugsweise wird noch mehr als der oben aufgeführte Perkolatwasserüberschuss über das Rezirkulationspumpwerk RZP1 oder den Perkolatwasserrezirkulationsschacht PSS2 aus dem Perkolatwasserbehälter P1 in den Perkolatwasserhälter P2 gefördert. Diese Mehrmenge muss ausgeglichen werden und zwar erfindungsgemäß mittels Rückführung dieser Mehrmenge von dem Perkolatwasserbehälter P2 zurück in den Perkolatwasserbehälter P1 über das Pumpwerk oder den Überlauf RZP2. Je höher diese Mehrmengenrezirkulation, je geringer die Perkolatwasserkonzentrationen im Perkolatwasserbehälter P1. Angestrebt werden sollte eine Mehmengemezirkulation, die zu einer Ammoniumkonzentration in dem Perkolatwasserbehälter P1 führt, die unterhalb der Hemmkonzentration der Biogasbildung liegt.

Die Rezirkulation des in der aeroben Biologie behandelten Perkolatwassers erfolgt aus dem Perkolatwasserbehälter P2 zurück in den Perkolatwasserbehälter P2 über die Leitung PRZ. Diese Rezirkulation kann jedoch auch über einen Bypass (BP2) an dem Perkolatwasserbehälter P2 vorbei erfolgen und somit direkt in die Perkolatwasserbeschickungsleitung der Fermenterkammer F1 - F6, die an den Perkolatwasserbehälter P2 angeschlossen ist. Damit wird die Auswaschintensität der betreffenden Fermenterkammer F1 - F6 intensiviert, da das gereinigte Perkolatwasser geringere Perkolatwasserkonzentrationen aufweist als das Perkolatwasser in dem Perkolatwasserbehälter P 2.

In Fig. 2 bedeuten:
F1 - F6: Fermenterkammern 1 - 6
P1, P2: Perkolatwasserbehälter 1 und 2
RZP1 und RZP2: Rezirkulationspumpwerke zwischen P1 und P2
PP1 und PP2: Perkolationspumpwerke
BP1 und BP2: Bypassleitungen um P2
P11 ... P26: Verschlussarmatur zur Steuerung der Beschickung der Fermenter aus P1 oder P2. Pxy bedeutet, dass die Fermenterkammer y aus dem Perkolatwasserbehälter x beschickt wird.
F11 ... F26: Verschlussarmatur zur Steuerung der Perkolatwasserrückführung aus den Fermenterkammern in P1 oder P2. Fxy bedeutet, dass Perkolatwasserrezirkulat aus der Fermenterkammer x in den Perkolatwassersammelschacht (PSS y) zur Rückführung in den Perkolatwasserbehälter y geführt wird.
PSS1, PSS2: Perkolatwasserrezirkulationsschächte.

Mit dieser neuartigen Verfahrenstechnik ergeben sich folgende Prozesscharakteristika:
1. Dem Perkolatwasserbehälter P 2 (oder auch wahlweise der aktuell mit diesem Perkolatwasserbehälter verbundenen Fermenterkammer) wird kontinuierlich gereinigtes Perkolatwasserrezirkulat aus dem Ablauf/Überlauf des Dortmundbrunnens zugeführt, Teilstrom 2, wahlweise mit geringen oder ganz ohne Belebtschlammanteile. Gleichzeitig wird diesem Perkolatwasserbehälter P 2 Perkolatwasserrezirkulat über einen Zeitraum von einer Woche aus der Fermentationskammer F 2 zugeführt, dessen Vergärungsprozess am weitesten fortgeschritten ist, und dessen Perkolatwasser somit schon weitgehend ausgegoren ist. Im Verlaufe dieser Woche wird aus der Fermenterkammer F 2 das Rohperkolatwasser ausgewaschen und gegen gereinigtes Perkolatwasser ausgetauscht. Im Verlaufe dieser Woche schreitet der Auswaschprozess immer weiter fort, bis nicht nur das Perkolatwasser als solches ausgewaschen ist, sondern auch weitere Organikbestandteile des Bioabfalles ausgewaschen werden. Dieser Auswaschprozess kann beliebig intensiviert werden, beispielsweise durch Erhöhung der Rezirkulationsrate, je nach gewünschtem / gefordertem Auswaschgrad.

Das Perkolatwasserrezirkulat wird vorzugsweise dann nicht mehr dem Perkolatwasserbehälter P2 zugeführt, wenn es einen Reinigungsgrad erreicht hat, der zu keiner weiteren Biogasbildung im Perkolatwasserbehälter P2 führt. Ab dann sollte dieses Perkolatwasserrezirkulat über die Bypassleitung BP 1 (siehe Fig. 2) direkt in den Zulauf der aeroben Biologie geführt werden.

Durch die kontinuierliche Rezirkulation zwischen anaerober Vergärung und aerober Perkolatwasserreinigung kann die Perkolatwasserorganik auf nahezu jeden gewünschten Abbaugrad reduziert werden, abgesehen von den minimalen Mengen an organischen Bestandteilen, die weder anaerob noch aerob abgebaut werden können. Diese Reduzierung der Restorganik im Bioabfall kann daher so weitgehend erfolgen, dass die MBA/TA Siedlungsabfall Deponiekriterien eingehalten werden können.
2. Das behandelte Perkolatwasser kann auf Indirekteinleiterqualität gereinigt werden. Da die Fließgleichgewichtskonzentration an CSB und NH₄-N im Perkolatwasserbehälter P2 um ein vielfaches geringer sind als im Perkolatwasserbehälter P1, werden sich in der aeroben Nachbehandlungsstufe Belebtschlammpopulationen etablieren, die insbesondere adaptiert sind für den Abbau der schwer abbaubaren CSB Fraktionen. Ein weiterer Vorteil dieser Konzeption besteht darin, dass besonders hochkonzentrierte Chargen aus dem Perkolatwasserbehälter P1 in dem Perkolatwasserbehälter P2 stark verdünnt werden, und damit nur stark abgepuffert auf die aerobe Nachbehandlung "durchschlagen".

Das aus dem Nitrifikationsbecken 12 entnommene, gereinigte Abwasser ist daher für optional nachfolgende Weiterbehandlungsstufen wie Ultrafiltration und Umkehrosmose sehr gut geeignet. Eine nachfolgende Umkehrosmosestufe kann das Abwasser auf Direkteinleiterqualität reinigen.
3. Der so behandelte Bioabfall wird nach Entleerung einer der Fermenterkammern F1 - F6 kein oder nur wenig Methangas in die Atmosphäre emittieren und eventuell noch anfallendes Sickerwasser wird sehr gering belastet sein.

## Patentansprüche

1. Verfahren zur Behandlung von bei der Trockenvergärung anfallendem Perkolatwasser unter Verwendung
- einer Mehrzahl von Fermentationskammern (F1 - F6),
- eines mit der Mehrzahl von Fermentationskammern (F1 - F6) verbundenen ersten Perkolatwasserbehälters (P1),
- eines mit der Mehrzahl von Fermentationskammern (F1 - F6) verbundenen zweiten Perkolatwasserbehälters (P2),
- eines Denitrifikationsbeckens (10),
- eines Denitrifikationsbeckens (10) nachgeschalteten Nitrifikationsbecken und
- eines Absetzbeckens (16), das einerseits mit dem Nitrifikationsbecken (12) und andererseits mit dem Denitrifikationsbecken (10) verbunden ist und einen Ablauf/Überlauf aufweist,
wobei
- der erste Perkolatwasserbehälter (P1) und/oder der zweite Perkolatwasserbehälter (P2) mit dem Denitrifikationsbecken (10) und
- der Ablauf/Überlauf des Absetzbeckens (16) mit dem zweiten Perkolatwasserbehälter (P2) und/oder mit der Mehrzahl von Fermentationskammern (F1-F6)
verbunden ist,
mit den Schritten;
- zeitlich versetztes Beschicken der Mehrzahl von Fermentationskammern (F1 - F6),
- Rezirkulieren von in einer Mehrzahl erster Fernrxentationskammern (F1 - F5) anfallendem Perkolatwasser zwischen der Mehrzahl erster Fermentationskammern (F1 - F5) und dem ersten Perkolatwasserbehälter (P1)
- Rezirkulieren von in einer weiteren Fermentationskammer (F6) anfallendem Perkolatwasser zwischen der weiteren Fermentationskammer (F6) und dem zweiten Perkolatwasserbehälter (P2), wobei die Trockenvergärung in der weiteren Fermentationskammer (F6) gegenüber der Mehrzahl erster Fermentationskammern (F1 - F5) am weitesten fortgeschritten ist,
- Beschicken des Denitriflkationsbeckens (10) mit Perkolatwasser aus dem ersten Perkolatwasserbehälter (P1) oder dem zweiten Perkolatwasserbehälter (P2) zur Reinigung des Perkolatwassers,
- Fördern von Belebtschlamm aus dem Nitrifikationsbecken in das Denitrifikationsbecken über das mit dem Denitrifikationsbecken einerseits und dem Nitrifikationsbecken andererseits verbundene Absetzbecken.
- Weiterleiten von denitrifiziertem Perkolatwasser aus dem Denitrifikationsbecken in das Nitrifikationsbecken über das zwischengeschaltete Absetzbecken; und
- Abführen von gereinigtem Perkolatwasser aus dem Ablauf/Überlauf des Absetzbeckens (16) und Zuführen des gereinigten Perkolatwassers in den zweiten Perkolatwasserbehälter (P2) oder in die aus dem zweiten Perkolatwasserbehälter (P2) beschickte weitere Fermentationskammer (F6).

2. Verfahren nach Anspruch 1, **gekennzeichnet durch** Pumpen des Überschussschlamms aus dem Absetzbecken (16) in einen der Perkolatwasserbehälter (P1, P2).

3. Verfahren nach einem der vorangehenden Ansprüche, **gekennzeichnet durch** Zuführen des zwischen dem zweiten Perkolatwasserbehälter (P2) und der weiteren Fermentationskammer (F6) geführten Perkolatwasserrezirkulats in den Zulauf einer aeroben Biologie, wenn das Perkolatwasser einen Reinigungsgrad erreicht hat, der zu keiner weiteren Biogasbildung in dem Perkolatwasserbehälter (P2) führt.

## Claims

1. A method for treating percolate water produced during dry fermentation, using
- a plurality of fermentation chambers (F1 - F6),
- a first percolate water container (P1) connected to the plurality of fermentation chambers (F1 - F6),
- a second percolate water container (P2) connected to the plurality of fermentation chambers (F1 - F6),
- a denitrification tank (10),
- a nitrification tank arranged downstream from the denitrification tank (10), and
- sedimentation tank (16) that is connected on the one hand to the nitrification tank (12) and on the other hand to the denitrification tank (10) and exhibits an outlet/overflow,
there being connected
- the first percolate water container (P1) and/or the second percolate water container (P2) to the denitrification tank (10) and
- the outlet/overflow of the sedimentation tank (16) to the second percolate water container (P2) and/or the plurality of fermentation chambers (F1-F6),
and having the steps:
- feeding the plurality of fermentation chambers (F1 - F6) temporally staggered,
- recirculating percolate water produced in the plurality of first fermentation chambers (F1 - F5), between the plurality of first fermentation chambers (F1-F5) and the first percolate water container (P1),
- recirculating percolate water produced in a further fermentation chamber (F6), between the further fermentation chamber (F6) and the second percolate water container (P2), dry fermentation in the further fermentation chamber (F6) having progressed furthest relative to the plurality of first fermentation chambers (F1 - F5),
- feeding the denitrification tank (10) with percolate water from the first percolate water container (P1) or the second percolate water container (P2) for purifying the percolate water,
- conveying activated sludge from the nitrification tank into the denitrification tank via the sedimentation tank connected on the one hand to the denitrification tank and on the other hand to the nitrification tank,
- passing on denitrified percolate water from the denitrification tank into the nitrification tank via the intermediate sedimentation tank; and
- discharging purified percolate water from the outlet/overflow of the sedimentation tank (16) and feeding the purified percolate water into the second percolate water container (P2) or into the further fermentation chamber (F6) that is fed by the second percolate water container (P2).

2. The method according to Claim 1, **characterized by** pumping the excess sludge from the sedimentation tank (16) into one of the percolate water containers (P1, P2).

3. The method according to one of the preceding claims, **characterized by** feeding the percolate water recirculate carried between the second percolate water container (P2) and the further fermentation chamber (F6) into the inlet of an anaerobic biology if the percolate water has achieved a degree of purifying that does not lead to further formation of biogas in the percolate water container (P2).

## Revendications

1. Procédé pour le traitement de l'eau de percolateur se produisant lors de la fermentation sèche en utilisant
- une multiplicité de chambres de fermentation (F1 - F6),
- un premier réservoir d'eau de percolateur (P), relié à la multiplicité de chambres de fermentation (F1 - F6),
- un deuxième réservoir d'eau de percolateur (P), relié à la multiplicité de chambres de fermentation (F1- F6),
- un bassin de dénitrification (10),
- un bassin de nitrification placé en aval du bassin de dévitrification (10) et
- un bassin sédimentaire (16), qui est connecté d'une part au bassin de nitrification (12) et d'autre part au bassin de dénitrification (10) et qui présente un écoulement/débordement,
où
- le premier réservoir d'eau de percolateur (P1) et/ou le deuxième réservoir d'eau de percolateur est connecté (P2) au bassin de dénitrification (10) et
- l'écoulement/débordement du bassin sédimentaire (16) est connecté au deuxième réservoir d'eau de percolateur (P2) et/ou à la multiplicité de chambres de fermentation (F1-F6)
avec les étapes :
- l'alimentation, décalée dans le temps, de la multiplicité de chambres de fermentation (F1 - F6),
- recirculation de l'eau de percolateur se produisant dans une multiplicité de premières chambres de fermentation (F1 - F5) entre la multiplicité de premières chambres de fermentation (F1- F5) et le premier réservoir d'eau de percolateur (P1),
- recirculation de l'eau de percolateur se produisant dans une autre chambre de fermentation (F6) entre l'autre chambre de fermentation (F6) et le deuxième réservoir d'eau de percolateur (P2), où la fermentation sèche dans l'autre chambre de fermentation (F6) est le plus avancée par rapport à la multiplicité de premières chambres de fermentation (F1 - F5),
- alimentation du bassin de dénitrification (10) avec de l'eau de percolateur du premier réservoir d'eau de percolateur (P1) ou le deuxième réservoir d'eau de percolateur (P2) pour nettoyer l'eau de percolateur,
- transport de boue activée du bassin de nitrification dans le bassin de dénitrification via le bassin sédimentaire, connecté au bassin de dénitrification d'une part et au bassin de nitrification d'autre part,
- transfert de l'eau de percolateur dénitrifiée du bassin de dénitrification dans le bassin de nitrification via le bassin sédimentaire intercalé ; et
- évacuation de l'eau de percolateur nettoyée de l'écoulement/débordement du bassin sédimentaire (16) et transport de l'eau de percolateur nettoyée dans le deuxième réservoir d'eau de percolateur (P2) ou dans l'autre chambre de fermentation (F6), alimentée par le deuxième réservoir d'eau de percolateur (P2).

2. Procédé d'après revendication 1, **caractérisé par** le pompage des boues en excès du bassin sédimentaire (16) dans l'un des réservoirs d'eau de percolateur (P1, P2).

3. Procédé d'après l'une des revendications précédentes, **caractérisé par le fait que** par l'alimentation du produit de la recirculation de l'eau du percolateur, entre le deuxième réservoir d'eau de percolateur (P2) et l'autre chambre de fermentation (F6) dans le conduit d'alimentation d'une biologie aérobe, lorsque l'eau de percolateur a atteint un degré d'épuration, qui n'entraîne pas d'autre formation de biogaz dans le réservoir d'eau de percolateur (P2).
